# FASCICULE DE BREVET EUROPEEN

(11) **EP 0 804 920 B1**
(45) Date de publication et mention de la délivrance du brevet: **02.06.2004**
(21) Numéro de dépôt: 97450005.0
(22) Date de dépôt: 18.04.1997
(51) Int. Cl.: A61K 7/16, A23K 1/18

(54) **Composition à effet antitartre et son utilisation dans des compléments alimentaires pour animaux**
Zusammensetzung gegen Zahnstein und seine Verwendung in Tierfutterzusatzstaioffen
Anticalculus composition and its use in animal feed supplements

(30) Priorité: 19.04.1996 FR 9604956
(43) Date de publication de la demande: 05.11.1997
(73) Titulaire: VIRBAC, 06516 Carros (FR)
(72) Inventeur: Cyr, Jean-Paul, M., 86490 Beaumont (FR); Denoun, Jean-Marc, M., 75016 Paris (FR)
(74) Mandataire: Joseph, Bernard

(56) Documents cités:
- EP-A- 0 173 568
- EP-A- 0 277 383
- EP-A- 0 366 869
- WO-A-93/11748
- WO-A-94/05251
- WO-A-94/08559
- WO-A-95/21605
- US-A- 5 405 836
- DATABASE WPI Week 9405 Derwent Publications Ltd., London, GB; AN 94-037343 XP002022212 & HU 208 481 B (REANAL FINOMVEGYSZERGYAR) , 29 novembre 1993

## Description

La présente invention se situe dans le domaine de la lutte contre le tartre, en particulier chez les animaux domestiques tels que les chiens.

L'invention concerne plus particulièrement une composition à effet antitartre permettant de lutter contre la formation de tartre ainsi que son utilisation en combinaison avec un complément alimentaire ou un article masticable pour animaux. L'invention concerne encore des compléments alimentaires et des articles masticables pour animaux ayant un effet antitartre.

La formation de tartre, en particulier chez les chiens, peut favoriser le développement de périodontites ayant notamment des conséquences sur la qualité et l'état de l'émail et/ou des gencives ou encore au niveau cardio-vasculaire.

Pour lutter contre le tartre et ses effets néfastes chez les animaux domestiques, le maître fait souvent appel au vétérinaire. Parallèlement, lorsque l'on veut éviter ou limiter ce type de consultation, on connait des comprimés à effet antitartre.

Cependant, les chiens croquent souvent ces comprimés dès qu'on les leur donne. Par conséquent, ils ne les mastiquent pas et ne les gardent pas assez longtemps dans leur gueule pour atteindre une efficacité acceptable.

Cela peut en outre conduire à une consommation importante et excessive de ces comprimés sans pouvoir obtenir l'effet recherché ou, pour le moins, sans pouvoir obtenir un tel effet de manière satisfaisante.

D'autres formes sont envisageables telles que par exemple les dentifrices.

Toutefois, d'un point de vue pratique, ce type de produit est difficile à utiliser dans le cas d'animaux et implique une intervention du maître qui peut se révéler bien délicate ou encore te recours à un service spécialisé de toilettage pour chiens.

On connaît également des objets masticables pour chiens qui, par leur mastication, présentent un effet abrasif dû à l'afflux de salive pouvant empêcher le tartre de se déposer en trop grande quantité.

Néanmoins, l'efficacité de ces objets masticables est limitée et ils ne permettent pas de détruire le tartre déjà formé de manière satisfaisante.

Aussi, il apparaît nécessaire de trouver de nouveaux moyens pour lutter contre le tartre et ses effets néfastes chez les animaux et en particulier chez les chiens, moyens qui permettraient de prévenir ou, pour le moins, de limiter le développement de maladies qui peuvent y être liées, en particulier le développement de périodontites.

En outre, ce besoin apparaît de manière de plus en plus importante dans la mesure où le nombre d'animaux de compagnie ne cesse de croître, tout particulièrement en ce qui concerne les chiens.

Le document US-5.405.836 décrit un produit pour l'hygiène buccale des animaux de compagnie, sous forme d'aliments, contenant des sels de zinc, visant à lutter contre la mauvaise haleine. Ces seuls de zinc agissent chimiquement sur les composés volatils sulfureux qui sont la cause de la mauvaise haleine. Bien que destiné aux animaux, le produit décrit ne vise pas à lutter contre le tartre.

Le document WO-94.08559 décrit un produit pour l'hygiène buccale de l'homme ayant une action antimicrobienne. Les bactéries présentes dans la bouche favorisant l'apparition de la plaque dentaire, la composition vise le traitement desdites bactéries pour éviter la formation de la plaque dentaire.

Bien qu'évoquant la réduction de la plaque dentaire, la composition proposée ne répond aux contraintes particulières liées aux produits pour l'hygiène buccale des animaux, bien différentes dé celles existant dans les applications chez l'homme.

Aussi, la présente invention a pour objectif de fournir une composition à effet antitartre permettant de lutter contre le tartre de manière efficace.

Elle a également pour objectif de fournir des compléments alimentaires ou autres articles masticables à effet antitartre particulièrement efficaces, dont la forme ne présente pas les inconvénients précités et qui puissent être utilisés chez les animaux de compagnie de manière simple et efficace.

Elle a encore pour objectif de fournir de tels compléments alimentaires et articles masticables qui n'impliquent pas une intervention importante et difficile du maître.

L'invention vise plus particulièrement des compléments et articles masticables destinés aux chiens.

A cette fin, la présente invention a pour objet une composition à effet antitartre caractérisée en ce qu'elle comprend :
- 0,5 à 5% en poids d'au moins un composé choisi parmi le silicate de zirconium et la silice hydratée ;
- 0,5 à 5% en poids d'au moins un composé choisi parmi le digluconate de chlorhexidine et la digluconate de zinc ;
- 0,5 à 5% en poids d'au moins un composé choisi parmi le thiocyanate de potassium, la glucose oxydase, la lysozyme et la lactoperoxydase ;
- 1 à 5% en poids d'au moins un composé acide choisi parmi la vitamine C et l'acide citrique.

L'invention a encore pour objet des compléments alimentaires à effet antitartre pour animaux comprenant la composition précitée.

Elle a encore pour objet des articles masticables à effet antitartre pour animaux domestiques comprenant ladite composition.

La présente invention a pour objet une composition à effet antitartre destinée à lutter contre le tartre, tout particulièrement chez les animaux domestiques.

Selon l'invention, l'expression "lutter contre le tartre" signifie éviter ou limiter la formation de tartre, mais également détruire le tartre déjà formé.

Les pourcentages sont exprimés en poids par rapport au poids total de la composition.

La composition conforme à l'invention peut comprendre un ou plusieurs autres ingrédients complémentaires tels que des dérivés fluorés.

Le ou les dérivés fluorés sont choisis de préférence parmi le monofluorophosphate de sodium et le fluorure de sodium. La composition comprend de préférence 0,2 à 5% en poids de monofluorophosphate de sodium et 0,05 à 1% en poids de fluorure de sodium.

Une composition préférée selon l'invention est la suivante :
- 0,5 à 5% en poids de silicate de zirconium ;
- 0,5 à 5% en poids de digluconate de chlorhexidine ;
- 0,5 à 5% en poids de thiocyanate de potassium ;
- 1 à 5% en poids de vitamine C ;
- 1 à 5% en poids d'acide citrique ;
- 0,2 à 5% en poids de monofluorophosphate de sodium ;
- 0,05 à 1% en poids de fluorure de sodium.

La composition selon l'invention permet, parallèlement à son action sur le tartre, de combattre les mauvaises odeurs buccales. La composition précédemment définie peut être préparée par tout procédé connu. On peut notamment mélanger les différents constituants précités dans l'eau, des aliments ou tout autre liant. Selon un autre aspect de l'invention, la composition à efffet antitartre est avantageusement utilisée en combinaison avec un support masticable.

Par "support masticable" , on entend un support qui implique une certaine mastication favorisant l'afflux de salive et qui procure un temps de séjour dans la gueule de l'animal suffisant pour permettre une action efficace de la composition à effet antitartre selon l'invention.

On choisit de préférence un support présentant un certain caractère abrasif susceptible de se conjuguer à l'action de la composition à effet antitartre.

Bien entendu, la taille et la forme du support masticable pour la composition à effet antitartre de la présente invention sont adaptées en fonction du type d'animal destiné à être traité.

Selon un mode de réalisation préféré de l'invention, la composition est associée à un complément alimentaire.

Ce complément alimentaire présente avantageusement un certain caractère masticable qui se combine à l'effet de la composition précédemment décrite pour conduire à un résultat particulièremen satisfaisant.

Le complément alimentaire peut en particulier consister en un os aliment pour chien.

Cet os aliment est à base de protéines et de vitamines et est entièrement digestible. Il est sec et présente une certaine dureté ainsi qu'un caractère abrasif. Ce type d'os favorise la mastication du chien.

Pour obtenir le complément alimentaire, la composition à effet antitartre est incorporée aux ingrédients de base lors de la fabrication de l'os aliment.

En outre, de par sa forme, l'os ne peut être directement croqué ou avalé. Aussi, lorsque l'on donne l'os au chien, celui-ci est obligé de le ronger, ce qui améliore encore l'effet antitartre du complément alimentaire.

Le complément alimentaire peut constituer une friandise pour le chien.

La composition selon l'invention conjuguée à un tel os aliment permet à la fois d'éviter la formation de tartre et de détruire le tartre déjà formé.

L'intervention du maître est en outre très limitée puisqu'il est simplement nécessaire de donner au chien l'os aliment à effet antitartre.

La composition conforme à la présente invention peut également être associée à d'autres types de supports à mâcher, tels que par exemple peaux de buffle, jouets, notamment en matière plastique. Ces articles peuvent être imprégnés ou enduits d'une composition selon l'invention au cours de leur préparation ou à la fin de celle-ci, selon les cas.

Conformément à la présente invention, on adapte la quantité de complément alimentaire ou d'articles masticables à effet antitartre donnée à un animal considéré selon son état d'entartrement.

Le traitement à l'aide des compléments alimentaires ou autres articles à effet antitartre selon l'invention peut être pratiqué pour prévenir la formation de tartre ou détruire le tartre déjà formé, selon l'état d'entartrement de l'animal considéré.

Ce traitement peut également être complémentaire d'un détartrage mécanique classique effectué par un vétérinaire afin d'en prolonger les effets.

Il peut encore avantageusement être mis en oeuvre lorsqu'un détartrage est déconseillé par le vétérinaire en raison de l'état de santé ou de l'âge de l'animal.

L'invention va être décrite plus en détail à l'aide de l'exemple donné ci-après à titre illustratif.

### EXEMPLE

On réalise la composition à effet antitartre suivante :
- 1% en poids de silicate de zirconium ;
- 1 % en poids de digluconate de chlorhexidine ;
- 1% en poids de thiocyanate de potassium ;
- 1,5% en poids de vitamine C ;
- 1,5% en poids d'acide citrique ;
- 0,76% en poids de monofluorophosphate de sodium ;
- 0,10% en poids de fluorure de sodium.

Cette composition est combinée à la composition pour os aliment suivante :
- 32,56% en poids de caséine ;
- 32,56% en poids de farine de volaille ;
- 3,725% de gélatine.

Les différents constituants sont mélangés en présence d'eau. La proportion de composition antitartre est d'environ 7% en poids par rapport au poids total du mélange.

### ETUDE RELATIVE A L'ACTION SUR L'ETAT D'ENTARTREMEN ET/OU SUR LA PRÉVENTION DE L'APPARITION DE TARTRE.

On prépare à partir de cet exemple des os aliments à effet antitartre de différentes tailles adaptées en fonction des chiens auxquels ils sont destinés, soit des os de 40 g pour "petits chiens", soit de 70 g pour "gros chiens".

Les os précédemment préparés ont été testés sur 42 chiens sélectionnés parmi les races suivantes :
- Fox terrier
- Berger allemand
- Nizinny
- Coquer spagneul
- Terrier du Tibet
- Westie
- Caniche moyen
- Colley
- Caniche nain toy
- York
- Croiset basset
- Beauceron
- Caniche royal
- Pinscher
- Teckel
- Wippet
- Briard

Pour cette étude, on considère les quatre états d'entartrement suivants (stades 1 à 4) à partir d'un état considéré comme normal (stade 0) :

| **Stades d'entartrement** | |
|---|---|
| **STADES** | **ENTARTREMENT** |
| 4 | 100% |
| 3 | 75% |
| 2 | 50% |
| 1 | 25% |
| 0 | plus de tartre |

La quantité d'os donnée à chaque chien est de 8 os/mois/chien.

Les chiens sont examinés à raison de deux fois par mois pendant trois mois.

L'examen porte sur l'état des gencives et de la plaque dentaire ainsi que sur le stade d'entartrement.

Les résultats obtenus sont rassemblés dans les tableaux I et II suivants :

**TABLEAU I**

| **STADES D'ENTARTREMENT** | | | | | | |
|---|---|---|---|---|---|---|
| | **Nbre de chiens** | **STADES D'ENTRARTEMENT** | | | | |
| | | **4** | **3** | **2** | **1** | **0** |
| Examen initial | 42 | 28 | 7 | 2 | 3 | 2 |
| + 15 jours | 42 | 22 | 9 | 4 | 4 | 3 |
| + 30 jours | 42 | 18 | 7 | 6 | 6 | 5 |
| + 45 jours | 42 | 11 | 8 | 3 | 12 | 8 |
| + 60 jours | 42 | 7 | 11 | 5 | 10 | 9 |
| + 75 jours | 42 | 4 | 3 | 4 | 15 | 16 |
| + 90 jours | 42 | 1 | 0 | 3 | 12 | 26 |

**TABLEAU II**

| **TAUX D'AMELIORATION DU STADE D'ENTARTREMENT** | | |
|---|---|---|
| **STADES** | **EXAMEN INITIAL** | **FIN DE TEST** |
| 4 | 66,66% | 2,38% |
| 3 | 16,66% | 0% |
| 2 | 4,76% | 7,14% |
| 1 | 7,14% | 28,57% |
| 0 | 4,76% | 61,90% |

Une bonne appétence de l'os a été notée sans désordres digestifs importants.

Ces résultats montrent notamment que lorsque l'os est pris régulièrement à raison de 8 os par mois pendant la période "d'attaque" de trois mois, on obtient un effet significatif de diminution de l'état d'entartrement.

Des améliorations plus rapides ont été notées pour des chiens passant du stade 4 aux stades 2 ou 1 plus brutalement que d'autres.

Un seul animal est resté au stade 4 en raison du mauvais état de sa dentition (dents déchaussées).

La quantité d'os peut être de 2 par semaine le premier mois, 1 par semaine le deuxième mois et 2 par mois pour le troisième mois ... Toutefois, le nombre d'os doit être adapté suivant les animaux.

Une nette amélioration de l'haleine a également été observée dès la consommation des premiers os.

## Revendications

1. Composition à effet antitartre **caractérisée en ce qu'**elle comprend :
- 0,5 à 5% en poids d'au moins un composé choisi parmi le silicate de zirconium et la silice hydratée :
- 0,5 à 5% en poids d'au moins un composé choisi parmi le digluconate de chlorhexidine et le digluconate de zinc ;
- 0,5 à 5% en poids d'au moins un composé choisi parmi le thiocyanate de potassium, la glucose oxydase, la lysozyme et la lactoperoxydase ;
- 1 à 5% en poids d'au moins un composé acide choisi parmi la vitamine C et l'acide citrique.

2. Composition selon la revendication 1, **caractérisée en ce qu'**elle comprend en outre au moins un dérivé fluoré.

3. Composition selon la revendication 2, **caractérisée en ce qu'**elle comprend 0,2 à 5% en poids de monofluorophosphate de sodium et 0,05 à 1% en poids de fluorure de sodium.

4. Composition selon l'une quelconque des revendications précédentes, **caractérisée en ce qu'**elle comprend :
- 0,5 à 5% en poids de silicate de zirconium ;
- 0,5 à 5% en poids de digluconate de chlorhexidine ;
- 0,5 à 5% en poids de thiocyanate de potassium ;
- 1 à 5% en poids de vitamine C ;
- 1 à 5% en poids d'acide citrique ;
- 0,2 à 5% en poids de monofluorophosphate de sodium ;
- 0,05 à 1% en poids de fluorure de sodium.

5. Composition selon l'une quelconque des revendications précédentes, **caractérisée en ce qu'**elle comprend :
- 1% en poids de silicate de zirconium ;
- 1% en poids de digluconate de chlorhexidine ;
- 1% en poids de thiocyanate de potassium ;
- 1,5% en poids de vitamine C ;
- 1,5% en poids d'acide citrique ;
- 0,76% en poids de monofluorophosphate de sodium ;
- 0,10% en poids de fluorure de sodium.

6. Complément alimentaire à effet antitartre pour animaux, **caractérisé en ce qu'**il comprend une composition selon l'une quelconque des revendications 1 à 5.

7. Complément selon la revendication 6, **caractérisé en ce qu'**il est masticable.

8. Complément selon la revendication 7, **caractérisé en ce qu'**il consiste en un os aliment.

9. Article masticable à effet antitartre pour animaux, **caractérisé en ce qu'**il comprend une composition selon l'une quelconque des revendications 1 à 5 en combinaison avec un support masticable.

## Patentansprüche

1. Zusammensetzung gegen Zahnstein, **dadurch gekennzeichnet, dass** sie Folgendes enthält:
- 0,5 bis 5 Gewichtprozent von mindestens einer Verbindung, ausgewählt unter Zirkoniumsilikat und wasserhaltiger Kieselsäure;
- 0,5 bis 5 Gewichtprozent von mindestens einer Verbindung, ausgewählt unter Chlorhexidin-Diglukonat und Zink-Diglukonat;
- 0,5 bis 5 Gewichtprozent von mindestens einer Verbindung, ausgewählt unter Kalium-Thiocyanat, Glukoseoxidase, Lysozym und Lactoperoxydase;
- 1 bis 5 Gewichtprozent von mindestens einer Säureverbindung, ausgewählt unter Vitamin C und Zitronensäure.

2. Zusammensetzung nach Anspruch 1, **dadurch gekennzeichnet, dass** sie außerdem mindestens ein Fluorderivat enthält.

3. Zusammensetzung nach Anspruch 2, **dadurch gekennzeichnet, dass** sie 0,2 bis 5 Gewichtprozent Natrium-Monofluorphosphat und 0,05 bis 1 Gewichtprozent Natriumfluorid enthält.

4. Zusammensetzung nach einem der vorhergehenden Ansprüche, **dadurch gekennzeichnet, dass** sie Folgendes enthält:
- 0,5 bis 5 Gewichtprozent Zirkoniumsilikat;
- 0,5 bis 5 Gewichtprozent Chlorhexidin-Diglukonat;
- 0,5 bis 5 Gewichtprozent Kalium-Thiocyanat;
- 1 bis 5 Gewichtprozent Vitamin C;
- 1 bis 5 Gewichtprozent Zitronensäure;
- 0,2 bis 5 Gewichtprozent Natrium-Monofluorphosphat;
- 0,05 bis 1 Gewichtprozent Natriumfluorid.

5. Zusammensetzung nach einem der vorhergehenden Ansprüche, **dadurch gekennzeichnet, dass** sie Folgendes enthält:
- 1 Gewichtprozent Zirkoniumsilikat;
- 1 Gewichtprozent Chlorhexidin-Diglukonat;
- 1 Gewichtprozent Kalium-Thiocyanat;
- 1,5 Gewichtprozent Vitamin C;
- 1,5 Gewichtprozent Zitronensäure;
- 0,76 Gewichtprozent Natrium-Monofluorphosphat;
- 0,10 Gewichtprozent Natriumfluorid.

6. Tierfutterzusatzstoff gegen Zahnstein bei Tieren, **dadurch gekennzeichnet, dass** sie eine Zusammensetzung nach einem der Ansprüche 1 bis 5 enthält.

7. Zusatzstoff nach Anspruch 6, **dadurch gekennzeichnet, dass** er kaubar ist.

8. Zusatzstoff nach Anspruch 7, **dadurch gekennzeichnet, dass** er aus einem Futterknochen besteht.

9. Kaubarer Artikel gegen Zahnstein bei Tieren, **dadurch gekennzeichnet, dass** er eine Zusammensetzung nach einem der Ansprüche 1 bis 5 in Verbindung mit einem kaubaren Träger enthält.

## Claims

1. Composition having an anti-tartar effect, **characterized in that** it comprises:
- from 0.5 to 5% by weight of at least one compound selected from zirconium silicate and hydrated silica;
- from 0.5 to 5% by weight of at least one compound selected from chlorhexidine digluconate and zinc digluconate;
- from 0.5 to 5% by weight of at least one compound selected from potassium thiocyanate, glucose oxidase, lysozyme and lactoperoxidase;
- from 1 to 5% by weight of at least one acid compound selected from vitamin C and citric acid.

2. Composition according to Claim 1, **characterized in that** it additionally comprises at least one fluorinated derivative.

3. Composition according to Claim 2, **characterized in that** it comprises from 0.2 to 5% by weight of sodium monofluorophosphate and from 0.05 to 1% by weight of sodium fluoride.

4. Composition according to any one of the preceding claims, **characterized in that** it comprises:
- from 0.5 to 5% by weight of zirconium silicate;
- from 0.5 to 5% by weight of chlorhexidine digluconate;
- from 0.5 to 5% by weight of potassium thiocyanate;
- from 1 to 5% by weight of vitamin C;
- from 1 to 5% by weight of citric acid;
- from 0.2 to 5% by weight of sodium monofluorophosphate;
- from 0.05 to 1% by weight of sodium fluoride.

5. Composition according to any one of the preceding claims, **characterized in that** it comprises:
- 1% by weight of zirconium silicate;
- 1% by weight of chlorhexidine digluconate;
- 1% by weight of potassium thiocyanate;
- 1.5% by weight of vitamin C;
- 1.5% by weight of citric acid;
- 0.76% by weight of sodium monofluorophosphate;
- 0.10% by weight of sodium fluoride.

6. Feed supplement for animals having an anti-tartar effect, **characterized in that** it comprises a composition according to any one of Claims 1 to 5.

7. Supplement according to Claim 6, **characterized in that** it is masticable.

8. Supplement according to Claim 7, **characterized in that** it consists of a food bone.

9. Masticable article for animals having an anti-tartar effect, **characterized in that** it comprises a composition according to any one of Claims 1 to 5 in combination with a masticable support.
